Europäisches Patentamt

European Patent Office

Office européen des brevets

(11) Publication number: **0 190 711**
**B1**

# EUROPEAN PATENT SPECIFICATION

(45) Date of publication of patent specification: **26.04.89**

(21) Application number: **86101395.1**

(22) Date of filing: **04.02.86**

(51) Int. Cl.⁴: **C 07 K 15/00,** C 12 P 21/00, C 12 N 15/00, C 12 N 5/00, C 12 N 9/64, G 01 N 33/577, C 12 Q 1/38 // (C12P21/00, C12R1:91)

(54) Monoclonal antibodies to tissue plasminogen activator derived from human normal cells.

(30) Priority: **07.02.85 JP 20983/85**
**08.02.85 JP 21862/85**

(43) Date of publication of application:
**13.08.86 Bulletin 86/33**

(45) Publication of the grant of the patent:
**26.04.89 Bulletin 89/17**

(84) Designated Contracting States:
**BE CH DE FR GB IT LI NL SE**

(56) References cited:
**EP-A-0 100 982**
**EP-A-0 156 169**
**FR-A-2 528 451**

**CHEMICAL ABSTRACTS, vol. 101, no. 1, 2nd July 1984, page 453, abstract no. 5227t, Columbus, Ohio, US; G. PETTERSON et al.: "Monoclonal antibodies to plasminogen activator from human uterine tissue", & PROG. FIBRINOLYSIS 1983, 6, 191-4**

**Prog. Fibrinolysis, vol. 6 (1983), pp. 191-194**

**Hematology, 3rd. edition, pages 1266-1276**

(73) Proprietor: **KOWA COMPANY, LTD.**
**6-29, 3-chome Nishiki**
**Naka-ku Nagoya-shi Aichi-ken (JP)**

(72) Inventor: **Saino, Yushi**
**C/o No. 17-43, Noguchi-cho 2-chome**
**Higashimurayama-shi Tokyo (JP)**
Inventor: **Doi, Takeshi**
**C/o No. 17-43, Noguchi-cho 2-chome**
**Higashimurayama-shi Tokyo (JP)**

(74) Representative: **Werner, Hans-Karsten, Dr. et al**
**Deichmannhaus am Hauptbahnhof**
**D-5000 Köln 1 (DE)**

**Description**

Field of the invention

This invention relates to novel monoclonal antibodies having specificity to tissue plasminogen activator (hereinafter referred to t-PA) collected from a tissue cultured liquor of human normal tissue derived cells and to their use.

Background of the invention

Cell fusion between lymphatic cells and myelomatic cells derived from mammals, e.g., mice, rats, etc., has made it possible to produce hybrid cells capable of being proliferated in a test tube and being cloned (Kohler and Milstein, *Nature*, Vol. 256, pp. 495—497 (1975)).

Since such hybrid cells have a property of secreting a uniform antibody having predetermined specificity (i.e., a monoclonal antibody), attempts have been made to prepare hybrid cells capable of producing monoclonal antibodies against various hormones, proteins and the like and to utilize the monoclonal antibodies produced therefrom in various studies (E. Dale Servier et al., *Clinical Chemistry*, Vol. 27, No. 11, pp. 1797—1806 (1981)). However, no monoclonal antibody to t-PA derived from human normal cells has been known.

Plasminogen activators which have so far been put into practical use as thrombolytic agents include urokinase separated and purified from urine or a cultured liquor of kidney cells, and streptokinase collected from *Streptococci.*

However, since these conventional plasminogen activators are inferior in affinity to fibrin, they should be frequently administered in large amounts in order to obtain the required effect on treatment. Administration of large doses is known to cause side effects, such as internal hemorrhage. In other words, plasmin formed in the circulating blood by the plasminogen activator administered is bound to a plasmin inhibitor in the blood and rapidly inactivated. In order to obtain a desired therapeutic effect, it is, therefore, necessary to administer a large amount of the plasminogen activator to produce plasmin at a level higher than that of the plasmin inhibitor in the blood. However, production of a large amount of plasmin causes decomposition of fibrinogen, leading to induction of the side effect of a tendency to hemorrhage.

To the contrary, a plasminogen activator that can produce plasmin on fibrin clot because of its high affinity to fibrin would degrade fibrin at a small dose level without undergoing influences of the plasmin inhibitor in the circulating blood so that the activity to degrade fibrinogen would be weakened. Under these circumstances, it has been keenly desired to develop thrombolytic agents having high fibrin affinity, exhibiting high thrombolytic activity at small levels, and causing less side effects.

On the other hand, a plasminogen activator having high affinity to fibrin has recently been separated and purified from a tissue cultured liquor of human melanoma cells as described in Japanese Patent Application (OPI) No. 28009/82 (the term "OPI" used herein means "published unexamined application), and European Patent Application No. 41766A. However, since this plasminogen activator, being derived from tumor cells, has problems on antigenicity and carcinogenicity, such a plasminogen activator cannot be presented for practical use.

Further, t-PA having the following properties has recently been separated and purified from a tissue cultured liquor of human normal tissue derived cells, as disclosed in U.S. Patent 4,505,893 and European Patent Application No. 100982A.

    a) Molecular weight: 63,000±10,000

    b) Isoelectric point: 7.0 to 8.5

    c) Affinity to fibrin: present

    d) Affinity to Concanavalin A: present

    e) Optimum pH: 7 to 9.5

    f) Reactivity to anti-urokinase specific antibody: none

This plasminogen activator has extremely high affinity to fibrin, and is also free from the above-described disadvantages associated with the conventional plasminogen activators because it is separated from a tissue cultured liquor of human normal tissue derived cells. Therefore, such plasminogen activator can be a thrombolytic agent causing less side effects.

In addition, the above-described plasminogen activator derived from human melanoma cells cannot be stabilized by addition of human serum albumin (*Throm. Haemostas.*, Vol. 48, No. 3, pp. 294—296 (1982)); on the other hand, the aforesaid t-PA is advantageous in that it can be stabilized by the addition of human serum albumin, and is not inactivated by long-term preservation or freeze-drying.

G. Pettersson et al. described in "Monoclonal antibodies to plasminogen activator from human uterine tissue, Prog. Fibrinolysis 1983, 6, 191-4" the preparation of antibodies directed to plasminogen activator from human uterine tissue from human melanoma cells and from porcine heart tissue. These antibodies were obtained utilizing the method described by Köhler and Milstein, 1975, using mouse myeloma cells with spleen cells from a mouse immunized with the above mentioned antigens.

Processes for obtaining t-PA include a process in which a tissue cultured liquor using human normal tissue derived cells, such as cells derived from human embryonic kidney, intestines, lungs, heart, ureter, skin or foreskin, or the whole embryo, human placenta derived cells or cells derived from human kidney, intestines, lungs, thyroid gland, heart, ureter or skin, in an appropriate growth medium is purified by a combination of techniques commonly employed in protein chemistry, such as adsorption using carriers, ion-exchange, fractional precipitation, gel-filtration, electrophoresis and various types of affinity chromatography. However, not only because t-PA is present in the cultured liquor in a trace amount but also because various chromatographic techniques show only weak specific affinity to t-PA, it has been difficult to obtain high purity t-PA. Moreover, the recovery reached by the above-described purification process was unsatisfactory. Accordingly, it has been desired to develop a specific purifying process which realizes high recoveries of high purity t-PA.

On the other hand, development of a method for detecting t-PA with high sensitivity has also been demanded as a means for studying functional mechanism of t-PA or for confirming blood levels in the treatment of thrombosis, etc. At present, it has been reported that t-PA can be determined with a monoclonal antibody which is prepared by using a melanoma derived plasminogen activator as an antigen as disclosed in Japanese Patent Application (OPI) No. 5121/84 and British Patent Application No. 2,122,219A. Further, a kit for enzyme linked immunosorbent assay (ELISA), in which a polyclonal antibody of t-PA is employed, is commercially available (Biopool t-PA ELISA Kit, produced by Biopool Co., Ltd.). These methods, however, are still unsatisfactory in terms of sensitivity and accuracy, due to poor specificity to t-PA, and improvements have been strongly desired.

Summary of the invention

The present inventors have conducted extensive and intensive investigations in order to overcome the above-described various problems, and, as a result, succeeded in obtaining monoclonal antibodies specific to t-PA by cell fusion.

Particularly, it has now been found that t-PA can be purified at high purity in high yield through one step by the use of these monoclonal antibodies. The present invention has thus been completed based on this finding. Further, these monoclonal antibodies can be used in immunodiagnosis and the like. Furthermore, the uniform monoclonal antibodies can be obtained in a large quantity whenever necessary by cultivating the stock hybrid cells.

That is, the present invention relates to monoclonal antibodies X-21 and X-23 specific to t-PA collected from a tissue cultured liquor of human normal tissue derived cells, having the following properties:
   a) Molecular weight: 63,000±10,000
   b) Isoelectric point: 7.0 to 8.5
   c) Affinity to fibrin: present
   d) Affinity to Concanavalin A: present
   e) Optimum pH: 7 to 9.5
   f) Reactivity to anti-urokinase specific antibody: none
and to use of the same, the monoclonal antibody X-21 having the following properties:
   a) Molecular weight: 150,000±5,000
   b) IgG subclass: IgG1
   c) Isoelectric point: 6.7 to 7.0
   d) Site of binding to antigen: determinant other than a fibrin binding site and a fibrinolytic activity-manifestating site
   e) N-terminal amino acid sequence at L Chain and H Chain:

```
                        1                          10
         L Chain:  Asp-Phe-Val-Met-Thr-Gln-Ser-Pro-Ala-Thr-

                                              20
                   Leu-Ser-Val-Thr-Pro-Gly-Asp-Arg-Val-Ser-

                                              30
                   Leu-Ser-Cys-Arg-Ala-Ser-Gln-X-Ile-Ser-Asp-

                                         40
                   Tyr-Leu-His-Trp-Tyr-Gln-Gln-Lys-(Gly)


                        1                          10
         H Chain:  Asp-Val-Gln-Leu-Gln-Gln-Ser-Gly-Pro-Asp-

                                              20
                   Leu-Val-Lys-Pro-Ser-Gln-Ser-Leu-Ser-Leu-

                                              30
                   Thr-Cys-Thr-Val-Thr-Gly-Tyr-(Ser)-Ile-Thr-X-Gly-Tyr
```

wherein groups in the parentheses are assumed residual groups; and X represents an unidentified residual group; and the monoclonal antibody X-23 having the following properties:

    a) Molecular weight: 153,000±5,000
    b) IgG subclass: IgG1
    c) Isoelectric point: 6.6 to 6.9
    d) Site of binding to antigen: a fibrin binding site
    e) N-terminal amino acid sequence at L Chain and H Chain:

```
                          1                               10
        L Chain:   Asp-Ile-Lys-Met-Thr-Gln-Ser-Pro-Ser-Ser-

                                                          20
                   Met-Tyr-Ala-Ser-Leu-Gly-Glu-Arg-Val-Thr-

                                                          30
                   Ile-Thr-Cys-Lys-Ala-Ser-Gln-Asp-Ile-Asn-

                                                  40      42
                   Ser-His-Leu-X-Trp-Phe-Gln-Gln-Lys-Pro-Gly-Lys


                          1
        H Chain:   (Pca)-Ile-Gln-Leu-Val-Gln-Ser-Gly-Pro-

                          10
                   Glu-Leu-Lys-Lys-Pro-Gly-Glu-Thr-Val-Lys-

                          20
                   Ile-Ser-Cys-Lys-Ala-Ser-Gly-Tyr-Thr-Phe-

                          30                    34
                   Thr-Asn-Tyr-Gly-Met
```

wherein groups in the parentheses are assumed residual groups; X represents an unidentified residual group; and Pca represents a pyrrolidone carboxylic acid.

Brief description of the accompanying drawings

    Figures 1a and 1b are graphs showing a correlation between results of isoelectric focusing analysis and antibody activities of X-21 and X-23 monoclonal antibodies, respectively.

    Figures 2a and 2b are immuno-affinity chromatographic patterns obtained using X-21 and X-23 monoclonal antibodies, respectively, which show a correlation between absorbance ($A_{280}$) of each eluate fraction and t-PA activity.

    Figure 3 is a calibration line obtained by ELISA using monoclonal antibodies of the present invention.

    Figure 4 is a graph plotting the amount of t-PA in healthy persons' plasma.

Detailed description of the invention

    The monoclonal antibodies which can be obtained in the present invention include monoclonal antibody X-21 which does not bind to a fibrinolytic activity-manifesting site and a fibrin binding site of t-PA, and monoclonal antibody X-23 which binds to a fibrin binding site of t-PA. That is, there are obtained: (1) a monoclonal antibody (X-23) which specifically binds to a fibrin binding site to eliminate fibrin affinity of t-PA while retaining the fibrinolytic activity; and (2) a monoclonal antibody (X-21) which binds to a determinant other than a fibrinolytic activity-manifesting site and a fibrin binding site, such binding having no influence on either fibrinolytic activity or affinity to fibrin.

    By a combined use of the above-mentioned monoclonal antibodies specifically binding to different antigen determinants, a method of assaying t-PA with increased sensitivity can be provided.

    According to the present invention, monoclonal antibodies characterized by their specificity to human normal cell derived t-PA can be provided from a hybrid cell system.

    The process of cell fusion according to the present invention is described below in detail.

(a) Preparation of antibody-producing cells:

    Antibody-producing cells can be prepared in accordance with a known technique, i.e., a process comprising immunizing an animal with an antigen, t-PA, and recovering the antibody-producing cells from the animal.

    The animals to be immunized include mice, rats, rabbits, guinea pigs, sheep, horses, cattle, etc., and the antibody-producing cells to be used include splenocytes, cells of lymphatic glands, peripheral blood cells, and the like.

(b) Preparation of myeloma cells:

An origin of myeloma cells to be used for cell fusion is not particularly restricted, and cell strains of various animals, such as mice, rats, rabbits, human beings, etc., may be employed. The cell strain is preferably resistant to chemicals and should be such that unfused myeloma cells cannot survive in a selected medium but only the hybrid cells may be proliferated. Such a chemical-resistant strain can be prepared by known methods as described in, for example, *Idenshi Kumikae Jitsuyoka Gijutsu* (Technique for Practicing Genic Recombination) (hereafter merely referred to as *"Idenshi"*), No. 3, p. 308, "IV Hybridoma", published by SCIENCE FORUM CO. (1982). The most commonly employed cell strain is a 8-azaguanine-resistant strain. This strain is deficient in hypoxanthine guanine phosphoribosyl transferase and cannot grow in a hypoxanthine-aminopterin-thymidine (HAT) medium. Further, the cell strain to be used is preferably non-secretory. For example, the preferred includes $P_3/X_{63}$-Ag 8U1 ($P_3U1$), $P_3/X63$-Ag. 6.5.3, $P_3/NS1$-I-Ag 4-1 or Sp2/O-Ag 14 derived from mouse myeloma strain SP-1 or mouse myeloma strain MOPC-21, rat myeloma cells 210.RCY3.Ag1,2,3, and the like.

(c) Cell fusion:

Cell fusion is generally carried out by cultivating a mixture of 1 to $5 \times 10^7$ myeloma cells and 1 to $5 \times 10^8$ antibody-producing cells usually at a mixing ratio of from 1/4 to 1/10 in a medium, such as an Eagle's minimum essential medium (MEM), a Rosewell Park Memorial Institute (RPMI) 1640 medium, etc. As a fusion accelerator, polyethylene glycol (PEG) having an average molecular weight of from 1,000 to 6,000 is preferred, but viruses may be employed as well. PEG is usually used in concentrations of from 30 to 50% by weight.

(d) Selective growth of hybrid cells:

Cells after cell fusion are appropriately diluted with, for example, RPMI 1640 Medium containing 10 to 20 vol% fetal calf serum, and from $10^5$ to $10^6$ cells are inoculated per well of a microtitre plate. A selective medium, e.g., HAT Medium, is added to each well, followed by cultivation while appropriately exchanging the selective medium with the fresh one. When an 8-azaguanine-resistant strain is used as myeloma cells, all the unfused myeloma cells perish in HAT Medium by about the 10th day from the inoculation. Since the antibody-producing cells are normal cells, they cannot grow in vitro for a long period of time. Therefore, the cells that are growing on after the 10th to 14th day from the inoculation are all hybrid cells.

(e) Screening of antibody-producing hybrid cells:

A screening method of the hybrid cells is not particularly limited and may be any of known methods, as described in *Idenshi*, No. 4, p. 314. For example, a part of a supernatant liquor of the well in which the hybrid cells have grown is taken out and reacted with t-PA. Then, a second antibody labeled with an enzyme, a radioisotope, a fluorescent substance or a chemi-luminescent substance is reacted therewith to determine the amount labeled to thereby detect the presence of an anti-human tissue plasminogen activator antibody.

(f) Cloning:

Because there is a possibility that each well may contain two or more kinds of hybrid cells, the cells are subjected to cloning by a limiting dilution method or a like technique to obtain monoclonal antibody-producing hybrid cells, as described, for example, in *Idenshi*, No. 4, p. 314.

The monoclonal antibodies having the highest purity can be collected from a culture supernatant liquor obtained by cultivating the hybrid cells in an appropriate medium, e.g., RPMI 1640 Medium, containing about 10 vol% fetal calf serum.

The antibodies can also be obtained in a larger quantity by intraperitoneally administering a mineral oil, e.g., Pristane (2,6,10,14-tetramethylpentadecane; a product of Aldrich Co.) to an animal of the same descent as the animal from which the myeloma cells are derived, and then administering the hybrid cells thereto to thereby proliferate the hybrid cells in a large quantity in vivo. In this case, ascites-tumor is formed in 10 to 18 days, and an antibody is produced in the serum and the ascites in high concentration.

Purification of the anti-human tissue plasminogen activator monoclonal antibodies from the ascites can be carried out in accordance with a usual method of recovery of antibodies from the serum, for example, the method of Hudson et al., *Practical Immunology*, Blackwell Sc. Publ. (1976).

The titer measurement of t-PA as purified as an antigen above is carried out by the following procedures (this also applies to the experiments described herein below).

The measurement is effected by a plate method using urokinase as the standard and an agar-added fibrin plate prepared by using 95% clotable fibrinogen (plasminogen content: about 50 casein units/g-clotable protein) as a starting material. A t-PA solution is diluted with a 0.067 M tris-HCl buffer (pH 8.0) containing 1% gelatin, 0.1 M sodium chloride and 0.1% sodium nitride, and the concentration of the plasminogen activator solution exhibiting the same lyzing zone as that of 10 IU/ml of urokinase on the fibrin plate was taken as 10 U/ml.

Physiochemical properties of the monoclonal antibodies X-21 and X-23 of the present invention are as follows:

a) Molecular weight:

150,000±5,000 for monoclonal antibody X-21
153,000±5,000 for monoclonal antibody X-23

The molecular weight of the monoclonal antibody was estimated by SDS-polyacrylamide gel slab electrophoresis analysis. As a molecular weight marker, SDS-PAGE Standard Molecular Weight Marker (High) (myosin 200 K, β-galactosidase 116.25 K, phosphorylase B 92.5 K, bovine serum albumin 66.2 K and ovoalbumin 45 K; produced by Bio Rad Co.) was used.

b) IgG subclass:

IgG1 for both monoclonal antibodies X-21 and X-23

Agarose was added to a 1/15 M phosphate-buffered saline (pH 7.2) to a concentration of 1% and dissolved therein by boiling. Five milliliters of the solution were spread on a slide glass to solidify. Two holes of 3 mm in diameter were made at a 3 mm interval therebetween, and 15 μl each of the cultured liquor of the fused cells or an antiserum against a mouse antibody prepared from a goat (anti-mouse Ig) was put in the hole. After the slide glass was allowed to stand in a moist chamber at room temperature for 18 hours, formation of a precipitation line due to antigen-antibody reaction between the hole containing the cultured liquor and the hole containing anti-mouse Ig was observed.

c) Isoelectric point:

pI=6.7 to 7.0 for monoclonal antibody X-21
pI=6.6 to 6.9 for monoclonal antibody X-23

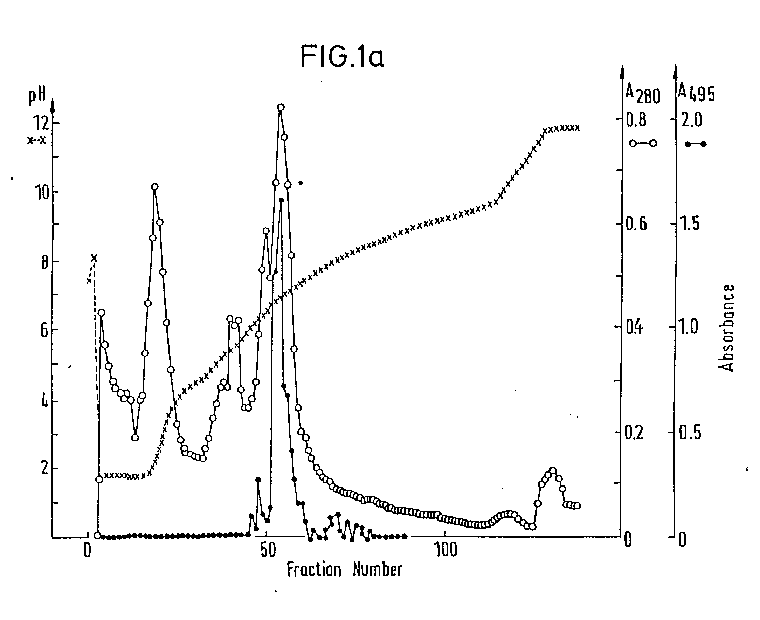

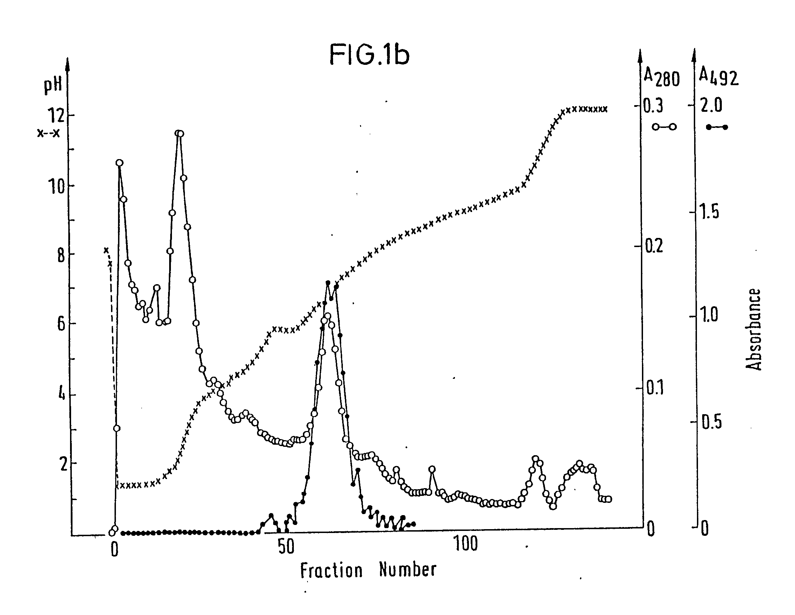

An apparatus for isoelectric focusing (produced by LKB) was used. About 1 mg of a partially purified monoclonal antibody was put in the apparatus, and an electric current was passed through the sample at a constant voltage of 700 V at 4°C for 40 to 60 hours in the presence of Ampholine, carrier ampholites of pH 3.5 to 9.5 (produced by LKB). When a steady state was reached, the sample was fractionated in 1 ml fractions. After pH measurement under ice-cooling, the protein concentration of the sample was determined by the absorbance at 280 nm, and the antibody specific to t-PA was detected by ELISA at the absorbance of 492 nm. The results obtained are shown in Figs. 1a and 1b.

d) Site of binding to antigen:

Antigen determinant other than fibrin binding site or fibrinolytic activity-manifesting site for monoclonal antibody X-21

Fibrin binding site for monoclonal antibody X-23

Each of monoclonal antibodies X-21 and X-23 was subjected to antigen-antibody reaction with t-PA, and the remaining fibrinolytic activity was assayed by a fibrin plate method. The results obtained are shown in Table 1 below.

TABLE 1

| Monoclonal antibody | Remaining activity |
| --- | --- |
| X-21 | 100% |
| X-23 | 25% |

It is confirmed from the results in Table 1 that monoclonal antibody X-21 does not bind to fibrinolytic activity-manifesting site of t-PA.

The above obtained reaction system was passed through a fibrin-Sepharose column prepared by the method of D. L. Heene et al. (*Thromb. Res.*, Vol. 2, pp. 137—154 (1973)), and eluted with physiological saline to which 0.3 M arginine hydrochloride had been added. The fibrinolytic activity of the eluate was determined by a fibrin plate method. The results obtained are as shown in Table 2.

TABLE 2

| Eluate | Fibrinolytic activity (%) | | |
| --- | --- | --- | --- |
| | t-PA alone | t-PA-X-21 | t-PA-X-23 |
| Passed Fractions | 20 | 23 | 86 |
| Eluate Fractions | 80 | 77 | 14 |

It is confirmed from the results in Table 2 that monoclonal antibody X-23 binds to fibrin binding site of t-PA whereas monoclonal antibody X-21 does not bind to fibrin binding site of t-PA.

e) N-terminal amino acid sequence at L Chain and H Chain:

Sequence analysis was conducted in accordance with an Edman method (Edman et al., *European J. Biochem., 1,* 80 (1967)) as follows:

A sample was introduced in a cartridge of a gaseous phase protein sequencer, Model 470A (produced by Applied Biosystems Co., Ltd.), and coupling, cleavage and conversion reactions were automatically effected using a previously prepared program. The resulting PTH (phenyl thiohydantoin)amino acid was dissolved in acetonitrile/$H_2O$/TFA (trifluoroacetic acid)=33/67/0.02 (by volume), and the solution was subjected to reversed phase high pressure liquid chromatography using an SEA-4 column (4.6$\phi \times$30 cm; produced by Senshu Kagaku K.K.). Each PTH-amino acid was identified by comparing the respective retention time with that of the standard PTH-amino acid.

The amino acid sequence of monoclonal antibody X-21 thus determined is as follows:

           1                 10

L Chain:  Asp-Phe-Val-Met-Thr-Gln-Ser-Pro-Ala-Thr-Leu-Ser-

              20

Val-Thr-Pro-Gly-Asp-Arg-Val-Ser-Leu-Ser-Cys-Arg-

              30                 40

Ala-Ser-Gln-X-Ile-Ser-Asp-Tyr-Leu-His-Trp-Tyr-Gln-Gln-Lys-(Gly)

            1                 10

H Chain:  Asp-Val-Gln-Leu-Gln-Glu-Ser-Gly-Pro-Asp-Leu-Val-

              20

Lys-Pro-Ser-Gln-Ser-Leu-Ser-Leu-Thr-Cys-Thr-Val-

              30

Thr-Gly-Tyr-(Ser)-Ile-Thr-X-Gly-Tyr

The amino acid sequence of monoclonal antibody X-23 thus determined is as follows:

            1                 10

L Chain:  Asp-Ile-Lys-Met-Thr-Gln-Ser-Pro-Ser-Ser-Met-Tyr-

              20

Ala-Ser-Leu-Gly-Glu-Arg-Val-Thr-Ile-Thr-Cys-Lys-

              30

Ala-Ser-Gln-Asp-Ile-Asn-Ser-His-Leu-X-Trp-Phe-Gln-

        40     42

Gln-Lys-Pro-Gly-Lys

            1                 10

H Chain:  (Pca)-Ile-Gln-Leu-Val-Gln-Ser-Gly-Pro-Glu-Leu-Lys-

              20

Lys-Pro-Gly-Glu-Thr-Val-Lys-Ile-Ser-Cys-Lys-Ala-

              30            34

Ser-Gly-Tyr-Thr-Phe-Thr-Asn-Tyr-Gly-Met

In the amino acid sequences, amino residues in the parentheses are assumed residual groups; X represents an unidentified residual group; and Pca represents a pyrrolidone carboxylic acid.

f) Cross reactivity to various plasminogen activators:

Reactivity to human urine derived urokinase or a swine heart extracted plasminogen activator was examined by ELISA (biotin-avidin system). It was confirmed that monoclonal antibodies X-21 and X-23 bind only to t-PA but do not bind to urokinase or a swine heart extracted plasminogen activator.

The anti-human tissue plasminogen activator monoclonal antibodies X-21 and X-23 of the present invention can be utilized for purification of t-PA by immuno-affinity chromatography using a solid type immuno-adsorbent to which the monoclonal antibody is chemically bound, such as cellulose, dextran, agarose, polyacrylamide, porous glass, etc. Specifically, a cultured liquor of human normal cells containing t-PA or a partially purified t-PA solution is brought into contact with the immuno-adsorbent packed in a column, whereby t-PA is adsorbed onto the immuno-adsorbent and held in the column. The immuno-adsorbent is then washed with a washing buffer at a pH of from 5 to 9 to remove any unadsorbed impurities. Thereafter, t-PA adsorbed onto the immuno-adsorbent is eluted out with an eluent. The monoclonal antibody is generally bound to the immuno-adsorbent in an amount of from 1 to 50 mg/ml, preferably from 3 to 10 mg/ml, using CNBr, periodic acid, an epoxide, a cross-linking agent, etc. The immuno-adsorbent, the method of binding the anti-human tissue plasminogen activator monoclonal antibody to the immuno-adsorbent, the eluent and the like which can be used herein are conventional, and any of those employed in general affinity chromatography may be employed.

Thus, impurities present in a tissue cultured liquor of human normal tissue derived cells or a crude t-PA solution can easily be separated and removed through one step by the use of the antibodies according to the present invention and, hence, extremely high purity t-PA can be obtained at high recoveries.

Since the above-described immuno-adsorbent can be repeatedly reused simply by washing with a washing buffer after desorption of t-PA, the t-PA purification procedure can be made easier and simpler, which ultimately leads to great advantages from the industrial viewpoint.

The anti-human tissue plasminogen activator monoclonal antibodies according to the present invention are also applicable to immuno-assay of t-PA, particularly preferably for EIA (enzyme immuno-assay) and ELISA. Introduction of the monoclonal antibodies into analysis of coagulation-lysis systems, such as studies on functional mechanism of t-PA in blood, enables trace analysis with high sensitivity.

The present invention is now illustrated in greater detail with reference to the following examples, but it should be understood that these examples do not limit the present invention. In these examples, all the percents and ratios are by weight, unless otherwise indicated.

Example 1

Purification of antigen:

Ammonium sulfate was added to 4 liters of a tissue cultured liquor of human embryonic kidney derived cells at a proportion of 300 g/l, followed by allowing the system to stand at 4°C overnight. The precipitate formed was collected by filtration and dissolved in a 1 M ammonium rhodanide solution containing 1 M sodium chloride. The resulting solution was 400 ml in liquid volume and had a t-PA activity of 21 U/ml and a specific activity of 10 $U/A_{280}$. The solution was adsorbed onto a phenyl-Sepharose column ($1\phi \times 10$ cm) and then eluted with ethylene glycol at a concentration gradient up to 50%. The eluate was 150 ml in volume and had a t-PA activity of 52 U/ml and a specific activity of 250 $U/A_{280}$.

The eluate was dialyzed against physiological saline containing 0.1 vol% Tween 80 (produced by Atlas Powder Co.), passed through an anti-urokinase IgG-Sepharose column and continuously adsorbed onto an arginine-Sepharose column ($1.5\phi \times 10$ cm). After thoroughly washing the column with a 0.5 M sodium chloride solution containing 0.1 vol% Tween 80, t-PA was eluted out with a 0.5 M arginine solution containing 0.1 vol% Tween 80. The resulting eluate was 52 ml in volume and had a t-PA activity of 98 U/ml and a specific activity of 3200 $U/A_{280}$.

The eluate was concentrated by freeze-drying and gel-filtration through a column ($1.5\phi \times 100$ cm) of Sephadex G-150 (produced by Pharmacia Co.) equilibrated with a 0.01 M phosphate buffer (pH 7.0) containing 1.5 M sodium chloride, 0.1 M arginine, 0.1 M EDTA and 0.1 vol% Tween 80 to collect active fractions. The solution thus collected was 15 ml in volume and had a t-PA activity of 270 U/ml and a specific activity of 12,500 $U/A_{280}$.

Immunization of mouse:

In a Freund's complete adjuvant was emulsified 100 μg of the thus purified t-PA, and the emulsion was subcutaneously administered three times to male BALB/c mice at two-week intervals for immunization. Of the immunized mice, the one having the highest serum antibody value to t-PA was intraperitoneally administered with 100 μg of t-PA to complete immunization.

Cell fusion:

The noted mouse was killed 3 days after the final immunization, and the spleen was excised, cut into pieces, filtered through a stainless steel mesh under pressure and suspended in MEM to prepare a splenocyte suspension. The splenocytes and mouse myeloma cells SP-1 were separately washed three times with serum-free MEM, and they were mixed at a proportion (number of cells) of 10/1. The mixture was centrifuged at 800 rpm for 5 minutes, and the precipitate was lightly untangled. One milliliter of a 44% polyethylene glycol (m.w.; 2,000) solution in MEM was gradually added thereto, and the mixture was subjected to cell fusion by gently rotating a centrifuge tube containing the mixture for 1 minute in warm water at 37°C. One minute later, 1 ml MEM was added to the system, followed by gentle rotation. MEM was further added thereto at a rate of 2 ml per minute. After a final volume of 10 ml was reached, the system was centrifuged at 1,000 rpm for 5 minutes, and the supernatant was removed. The resulting cell precipitate was suspended in RPMI 1640 Medium containing 10 vol% fetal calf serum (10% FCS) in such a concentration that $1\times10^6$ cells/ml of SP-1 were present. The suspension was inoculated in a 96-well microplate (NUNC Co.) in 0.1 ml portions.

After one day, 0.1 ml of RPMI 1640 Medium containing HAT (hypoxanthine $1\times10^{-4}$ M, aminopterin $4\times10^{-7}$ M and thymidine $1.6\times10^{-5}$ M)-10% FCS (HAT Medium) was added to each well. Thereafter, half the volume of the culture medium was replaced with fresh HAT Medium every 3 to 4 days to accelerate HAT selectivity. Growth of hybrid cells was observed in some wells from about the 7th day, and proliferation of the hybrid cells was noted on the 10th to 14th day in almost every well.

Screening of antibody-producing cells:

The cultured liquor was collected from the wells wherein the hybrid cells had grown, and the presence of the hybrid cells capable of producing an antibody to t-PA was confirmed by ELISA. ELISA was carried out by using a biotin-avidin system of Jean-Luc Guesdon *(J. Histochem. Cytochem.,* Vol. 27, p. 1131 (1979)). t-PA was put in each well of a 96-well microplate in an amount of 0.05 μg/100 μl/well, followed by allowing to stand at 25°C for 18 hours to thereby adsorb t-PA onto a solid phase. After washing three times with 200 μl portions of a phosphate buffer solution (PBS), 200 μl of PBS containing 0.5% bovine serum albumin (BSA) was added thereto, and the system was left to stand at 4°C overnight to block the unadsorbed portion in each well.

Then, 100 μl/well of the cultured liquor to be tested was placed in the well, followed by allowing to react at 37°C for 2 hours. After washing three times with PBS containing 0.05 vol% Triton X-100 (produced by Rohm & Haas Co.), 100 μl/well of biotinyl goat anti-mouse IgG (produced by EY Lab.) was added thereto, followed by allowing the system to react at 37°C for 1 hour. After washing, 100 μl/well of horseradish peroxide-avidin (produced by EY Lab.) was added to the well. One hour later, the system was washed with PBS-Triton, and a 0.1 M citric acid-sodium hydroxide buffer solution (pH 4.0) containing 0.001% hydrogen peroxide and 0.15 mg/ml of azino-bis(3-ethylbenzothiazoline-6,6-sulfonic acid) (produced by Nakarai Kagaku Yakuhin K.K.) was added thereto. The thus obtained sample was determined for absorbance at 414 nm. Among the test samples, color development was observed only in those wells wherein antibody against t-PA was present.

Cloning of monoclonal antibody-producing cells:

The hybrid cells in the wells of the microplate which were positive in ELISA were harvested and the number of cells was measured. After the cultured liquor was diluted with 10% FCS-added RPMI 1640 Medium, it was inoculated in a microtray at a rate of 0.5 cell per well in which $2\times10^5$ mouse celiac cells had been inoculated and cultured on the day before as feeder cells. The cultivation was carefully continued for about 2 weeks while occasionally exchanging the medium until a colony of hybrid cells was formed. The amount of antibody produced in each of the wells in which the hybrid cells had grown in colonies was measured by ELISA, and those hybrid cells which were positive in ELISA were again subjected to cloning.

There were obtained clone cells (hybridoma) X-21 and X-23 having high capability of producing an antibody; these were deposited at Institute for Fermentation, Osaka (IFO) on January 14, 1986 with the deposition Nos. IFO 50086 and IFO 50087, respectively.

Preparation of monoclonal antibody to t-PA in vivo:

A 7 or more-week-old BALB/c mouse was intraperitoneally administered with 0.5 ml of Pristane (produced by Aldrich Co.). After the elapse of 1 week or more, 1 to $9\times10^8$ cells/animal of X-21 or X-23 were intraperitoneally inoculated to mice. From the 7th day from the inoculation of X-21 or X-23, the body weight of the mice abruptly increased, reaching the peak on the 10th to 15th day. When the body weight reached around its peak, the mice were killed under ether anesthesia, and the ascites was taken out and centrifuged at 3,000 rpm for 10 minutes to obtain 5 to 15 ml/animal of monoclonal antibody-containing ascites.

Purification of monoclonal antibodies X-21 and X-23:

A monoclonal antibody was purified from 10 ml of the ascites as obtained using the clone cells X-21 [X-23]*, as follows (* hereinafter, the numerals in "[ ]" relate to the monoclonal antibody X-23).

To 10 ml of the ascites was added 10 ml [5 ml] of a saturated ammonium sulfate solution, and the system was allowed to stand at 4°C overnight. The formed precipitate was collected by centrifugation, dissolved in 5 ml [10 ml] of a 0.01 M sodium phosphate buffer solution (pH 8) and dialyzed against 100 times the volume of the same buffer overnight. The resulting solution was centrifuged at 10,000 rpm for 5 minutes, and the supernatant liquor was separated. This sample was passed through a column for 20 ml of DEAE Toyopeal 650 M (produced by Toyo Soda Manufacturing Co., Ltd.) equilibrated with a sufficient amount of a 0.01 M sodium phosphate buffer solution (pH 8). After washing with a 0.01 M phosphate buffer solution (pH 8) until $A_{280}$ fell to 0.02 or smaller, a monoclonal antibody was fractionally eluted out with the same phosphate buffer solution as used above with its concentration of sodium chloride being linearly increased up to 0.2 M. The active fractions containing the monoclonal antibody decided through antibody

9

activity by ELISA and SDS (sodium dodecyl sulfate)-polyacrylamide gel electrophoresis analysis were then fractionated using a Sephadex G-200 column (produced by Pharmacia Co.) equilibrated with a phosphate-buffered saline to obtain 106 mg of monoclonal antibody X-21 [73 mg of monoclonal antibody X-23].

Example 2
Purification of t-PA by immuno-affinity chromatography using monoclonal antibody X-21:

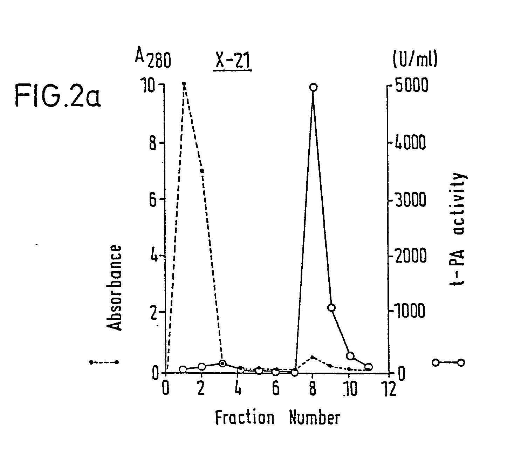

Ammonium sulfate was added to 5 liters of a human embryonic kidney tissue cultured liquor in a proportion of 300 g/l, and the system was allowed to stand at 4°C overnight. The precipitate formed was collected by centrifugation and dissolved in physiological saline containing 0.1 vol% Tween 80. The resulting solution was 480 ml in volume, and had an activity of 35 U/ml. This crude solution, was concentrated to about 1/50 of its original volume, and 5 ml of the concentrate was passed through a column packed with monoclonal antibody X-21 bound to Sepharose 4B (3 mg-antibody/ml-carrier) washed with a 0.02 M tris-HCl buffer solution containing 0.01 vol% Tween 80 and 1 M sodium chloride. After removing any unadsorbed portion by washing, t-PA adsorbed onto the column was eluted with 0.1 M glycine-HCl (pH 2.5) containing 50 vol% ethylene glycol and 0.01 vol% Tween 80. The absorbance at 280 nm and the t-PA activity in the eluted fractions are shown in Fig. 2a.

The properties of the crude solution and the eluted fractions are shown in Table 3 below.

TABLE 3

| Sample | Activity (U/ml) | Recovery (%) |
|---|---|---|
| Crude Solution | 7,800 | 100 |
| Eluted Fractions | 6,500 | 83 |

Example 3
Purification of t-PA by immuno-affinity chromatography using monoclonal antibody X-23:

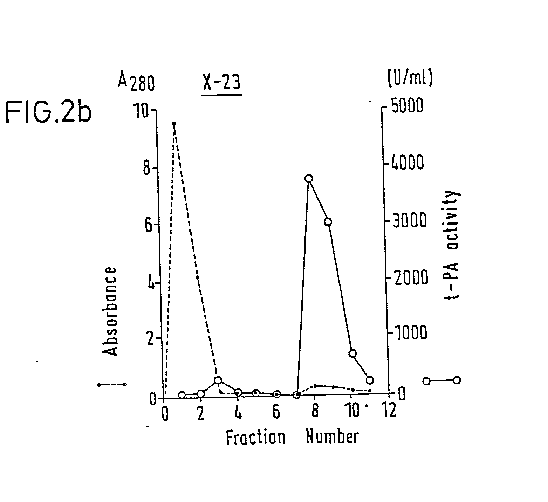

Ammonium sulfate was added to 5 liters of a human embyronic kidney tissue cultured liquor in a proportion of 300 g/l, and the system was allowed to stand at 4°C overnight. The precipitate formed was collected by centrifugation and dissolved and dialyzed against a 0.02 M tris-HCl buffer solution containing 0.1 vol% Tween 80 and 1 M sodium chloride. The resulting solution was 480 ml in volume, and had an activity of 35 U/ml. This crude solution was concentrated to about 1/50 of its original volume, and 5 ml of the concentrate was passed through a column packed with monoclonal antibody X-23 bound to Sepharose 4B (3 mg-antibody/ml-carrier) washed with a 0.02 M tris-HCl buffer solution containing 0.01 vol% Tween 80 and 1 M sodium chloride. After removing any unadsorbed portion by washing, t-PA adsorbed onto the column was eluted with 0.1 M glycine-HCl (pH 2.5) containing 50 vol% ethylene glycol and 0.01 vol% Tween 80. The absorbance at 280 nm and the t-PA activity in the eluted fractions are shown in Fig. 2b.

The properties of the crude solution and the eluted fractions are shown in Table 4 below.

TABLE 4

| Sample | Activity (U/ml) | Recovery (%) |
|---|---|---|
| Crude Solution | 7,800 | 100 |
| Eluted Fractions | 7,700 | 98 |

Example 4
Quantitative determination of t-PA by ELISA using monoclonal antibody

Reagents for ELISA were prepared using monoclonal antibodies X-21 and X-23 as obtained in Example 1.

(1) Preparation of horseradish peroxidase (HRP)-labeled monoclonal antibody-Fab'

Thirty milligrams of monoclonal antibody X-23 IgG were digested by pepsin and then subjected to gel-filtration using Sephadex G-100 to obtain an F(ab')$_2$ fraction. The following process was carried out in accordance with the method of S. Yoshitake et al., J. Biochem., 92, 1413 1424 (1982). To a solution of the resulting F(ab')$_2$ in a buffer solution was added β-mercaptoethylamine. After the solution was subjected to reduction treatment at 37°C for 2 hours, it was fractionated using Sephadex G-25 to collect Fab'. Maleimide-HRP and Fab' were mixed and reacted at 4°C for 20 hours, and the reaction mixture was purified by column chromatography using Sephacryl S-200 (produced by Pharmacia Co.) equilibrated with a 0.1 M sodium phosphate buffer solution (pH 6.5). Absorbances at 280 nm and 403 nm were measured, and the fractions around the absorption maximum were collected.

(2) ELISA System

Monoclonal antibody X-21 dissolved in a 0.05 M sodium carbonate was placed in wells of a 96-well microplate in an amount of 200 µl/well. After incubation at 25°C for 3 hours, the solution was discarded. PBS containing 0.05% Tween 20 was then added to each well, followed by shake-washing, and the solution was discarded. t-PA was dissolved in a PBS/Tween solution containing 0.1% BSA was added thereto in an amount of 200 µl/well, followed by reacting at 25°C for 16 hours. After washing with a PBS/Tween solution having the same composition as used above, 200 µl of HRP-labeled monoclonal antibody X-23-Fab' diluted with the PBS/Tween solution was added thereto, followed by reacting at 25°C for 3 hours. The solution was discarded, and, after washing, 200 µl of 0.1 M citric acid-0.2 M sodium phosphate buffer solution (pH 5.0) containing 0.01% hydrogen peroxide and 0.4 mg/ml of o-phenylenediamine was added thereto as an enzyme substrate, followed by enzymatic reaction at 25°C for 30 minutes. The reaction was terminated by adding 50 µl of a 4.5 M sulfuric acid, and the absorbance at 492 nm was measured.

(3) Sensitivity of assay system

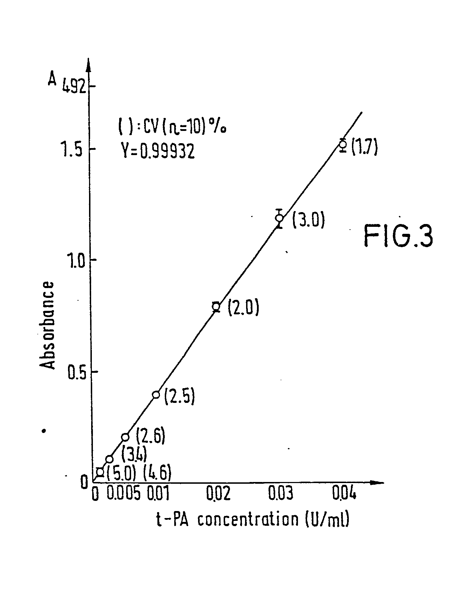

A calibration line was prepared from averages of 10 measurements using t-PA at various concentrations of from 0.04 to 0.00125 U/ml. The results are shown in Fig. 3.

From the results, it can be seen that t-PA having a concentration down to 0.002 U/ml can be quantitatively determined by the use of this assay system.

Example 5

Quantitative determination of t-PA in human plasma

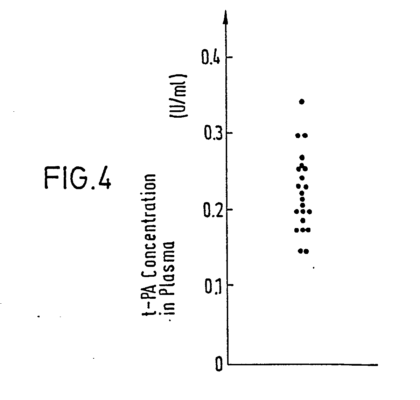

t-PA in human plasma was quantitatively determined using the ELISA system as prepared in Example 4. Plasma samples were prepared according to the method of Bergsdorf et al., *Thromb. Haemost.,* Vol. 50, pp. 740—744 (1983). Citrated human blood was centrifuged at 3,000 rpm for 15 minutes, and to 25 µl of the resulting blood plasma was added 25 µl of a 1 M sodium acetate buffer solution (pH 3.9), followed by allowing the system to stand at room temperature for 1 hour. To the system was added 50 µl of a 0.1 M disodium hydrogenphosphate-0.4 N sodium hydroxide aqueous solution for neutralization, and 400 µl of a PBS/Tween solution containing 0.1% BSA was added thereto to obtain a 20-fold diluted solution as a sample. Plasma samples were collected from 22 healthy persons. The results of t-PA determination are shown in Fig. 4.

These results show that the t-PA level in healthy persons' plasma averaged 0.222±0.011 (SD) U/ml (5.6 ng/ml).

While the invention has been described in detail and with reference to specific embodiments thereof, it will be apparent to one skilled in the art that various changes and modifications can be made within the claimed invention.

**Claims**

1. A monoclonal antibody, which is specific to a tissue plasminogen activator obtained from a tissue cultured liquor of human normal tissue derived cells, having the following properties:
   a) Molecular weight: 63,000±10,000
   b) Isoelectric point: 7.0 to 8.5
   c) Affinity to fibrin: present
   d) Affinity to Concanavalin A: present
   e) Optimum pH: 7 to 9.5
   f) Reactivity to anti-urokinase specific antibody: none
and which antibody has the following properties:
   a) Molecular weight: 150,000±5,000
   b) IgG subclass: IgG1
   c) Isoelectric point: 6.7 to 7.0
   d) Site of binding to antigen: determinant other than a fibrin binding site and a fibrinolytic activity-manifestating site
   e) N-terminal amino acid sequence at L Chain and H Chain:

                      1                            10

L Chain:  Asp-Phe-Val-Met-Thr-Gln-Ser-Pro-Ala-Thr-

                                       20

Leu-Ser-Val-Thr-Pro-Gly-Asp-Arg-Val-Ser-

                                     30

Leu-Ser-Cys-Arg-Ala-Ser-Gln-X-Ile-Ser-Asp-

                                   40

Tyr-Leu-His-Trp-Tyr-Gln-Gln-Lys-(Gly)

EP 0 190 711 B1

H Chain:  Asp-Val-Gln-Leu-Gln-Gln-Ser-Gly-Pro-Asp-  (1 ... 10)

Leu-Val-Lys-Pro-Ser-Gln-Ser-Leu-Ser-Leu-  (... 20)

Thr-Cys-Thr-Val-Thr-Gly-Tyr-(Ser)-Ile-Thr-X-Gly-Tyr  (... 30)

wherein groups in the parentheses are assumed residual groups; and X represents an unidentified residual group.

2. A monoclonal antibody as in claim 1, which is produced by cultivating a hybrid cell of myeloma cells of mice and splenocytes of mice immunized with a tissue plasminogen activator obtained from a tissue cultured liquor of human normal tissue derived cells, having the properties of claim 1.

3. A monoclonal antibody as in claim 2, wherein said hybrid cell in Hybridoma X-21 having IFO 50086.

4. A process for purifying a tissue plasminogen activator derived from human normal cells by immuno-affinity chromatography, which comprises using a monoclonal antibody which is specific to a tissue plasminogen activator obtained from a tissue cultured liquor of human normal tissue derived cells, having the properties of claim 1, and which antibody has the following properties:

a) Molecular weight: 150,000±5,000
b) IgG subclass: IgG1
c) Isoelectric point: 6.7 to 7.0
d) Site of binding to antigen: determinant other than a fibrin binding site and a fibrinolytic activity-manifestating site
e) N-terminal amino acid sequence at L Chain and H Chain:

L Chain:  Asp-Phe-Val-Met-Thr-Gln-Ser-Pro-Ala-Thr-  (1 ... 10)

Leu-Ser-Val-Thr-Pro-Gly-Asp-Arg-Val-Ser-  (... 20)

Leu-Ser-Cys-Arg-Ala-Ser-Gln-X-Ile-Ser-Asp-  (... 30)

Tyr-Leu-His-Trp-Tyr-Gln-Gln-Lys-(Gly)  (... 40)

H Chain:  Asp-Val-Gln-Leu-Gln-Glu-Ser-Gly-Pro-Asp-  (1 ... 10)

Leu-Val-Lys-Pro-Ser-Gln-Ser-Leu-Ser-Leu-  (... 20)

Thr-Cys-Thr-Val-Thr-Gly-Tyr-(Ser)-Ile-Thr-X-Gly-Tyr  (... 30)

wherein groups in the parentheses are assumed residual groups; and X represents an unidentified residual group.

5. A process for detecting a tissue plasminogen activator derived from human normal cells by immuno-assay, which comprises using a monoclonal antibody which is specific to a tissue plasminogen activator obtained from a tissue cultured liquor of human normal tissue derived cells, having the properties of claim 1, and which antibody has the following properties:

a) Molecular weight: 150,000±5,000
b) IgG subclass: IgG1
c) Isoelectric point: 6.7 to 7.0
d) Site of binding to antigen: determinant other than a fibrin binding site and a fibrinolytic activity-manifestating site
e) N-terminal amino acid sequence at L Chain and H Chain:

L Chain:  Asp-Phe-Val-Met-Thr-Gln-Ser-Pro-Ala-Thr-  (1 ... 10)

Leu-Ser-Val-Thr-Pro-Gly-Asp-Arg-Val-Ser-  (... 20)

12

                                    30
Leu-Ser-Cys-Arg-Ala-Ser-Gln-X-Ile-Ser-Asp-

                                    40
Tyr-Leu-His-Trp-Tyr-Gln-Gln-Lys-(Gly)


                 1                          10
H Chain:  Asp-Val-Gln-Leu-Gln-Glu-Ser-Gly-Pro-Asp-

                                    20
Leu-Val-Lys-Pro-Ser-Gln-Ser-Leu-Ser-Leu-

                                    30
Thr-Cys-Thr-Val-Thr-Gly-Tyr-(Ser)-Ile-Thr-X-Gly-Tyr

wherein groups in the parentheses are assumed residual groups; and X represents an unidentified residual group.

6. A monoclonal antibody which is specific to a tissue plasminogen activator obtained from a tissue cultured liquor of human normal tissue derived cells, having the properties of claim 1, and which antibody has the following properties:
    a) Molecular weight: 153,000±5,000
    b) IgG subclass: IgG1
    c) Isoelectric point: 6.6 to 6.9
    d) Site of binding to antigen: a fibrin binding site
    e) N-terminal amino acid sequence at L Chain and H Chain:


                 1                          10
L Chain:  Asp-Ile-Lys-Met-Thr-Gln-Ser-Pro-Ser-Ser-

                                    20
Met-Tyr-Ala-Ser-Leu-Gly-Glu-Arg-Val-Thr-

                                    30
Ile-Thr-Cys-Lys-Ala-Ser-Gln-Asp-Ile-Asn-

                                    40        42
Ser-His-Leu-X-Trp-Phe-Gln-Gln-Lys-Pro-Gly-Lys


                 1
H Chain:  (Pca)-Ile-Gln-Leu-Val-Gln-Ser-Gly-Pro-

                 10
Glu-Leu-Lys-Lys-Pro-Gly-Glu-Thr-Val-Lys-

                 20
Ile-Ser-Cys-Lys-Ala-Ser-Gly-Tyr-Thr-Phe-

                 30            34
Thr-Asn-Tyr-Gly-Met

wherein groups in the parentheses are assumed residual groups; X represents an unidentified residual group; and Pca represents a pyrrolidone carboxylic acid.

7. A monoclonal antibody as in claim 6, which is produced by cultivating a hybrid cell of myeloma cells of mice and splenocytes of mice immunized with a tissue plasminogen activator obtained from a tissue cultured liquor of human normal tissue derived cells, having the properties of claim 1.

8. A monoclonal antibody as in claim 7, wherein said hybrid cell is hybridoma X-23 having IFO 50087.

9. A process for purifying a tissue plasminogen activator derived from human normal cells by immuno-affinity chromatography, which comprises using a monoclonal antibody which is specific to a tissue plasminogen activator obtained from a tissue cultured liquor of human normal tissue derived cells, having the properties of claim 1, and which antibody has the following properties:
    a) Molecular weight: 153,000±5,000
    b) IgG subclass: IgG1
    c) Isoelectric point: 6.6 to 6.9

d) Site of binding to antigen: a fibrin binding site
e) N-terminal amino acid sequence at L Chain and H Chain:

$$\overset{1}{\phantom{}} \qquad\qquad\qquad \overset{10}{\phantom{}}$$

L Chain: Asp-Ile-Lys-Met-Thr-Gln-Ser-Pro-Ser-Ser-

$$\overset{20}{\phantom{}}$$

Met-Tyr-Ala-Ser-Leu-Gly-Glu-Arg-Val-Thr-

$$\overset{30}{\phantom{}}$$

Ile-Thr-Cys-Lys-Ala-Ser-Gln-Asp-Ile-Asn-

$$\overset{40}{\phantom{}} \quad \overset{42}{\phantom{}}$$

Ser-His-Leu-X-Trp-Phe-Gln-Gln-Lys-Pro-Gly-Lys

$$\overset{1}{\phantom{}}$$

H Chain: (Pca)-Ile-Gln-Leu-Val-Gln-Ser-Gly-Pro-

$$\overset{10}{\phantom{}}$$

Glu-Leu-Lys-Lys-Pro-Gly-Glu-Thr-Val-Lys-

$$\overset{20}{\phantom{}}$$

Ile-Ser-Cys-Lys-Ala-Ser-Gly-Tyr-Thr-Phe-

$$\overset{30}{\phantom{}} \qquad \overset{34}{\phantom{}}$$

Thr-Asn-Tyr-Gly-Met

wherein groups in the parentheses are assumed residual groups; X represents an unidentified residual group; and Pca represents a pyrrolidone carboxylic acid.

10. A process for detecting a tissue plasminogen activator derived from human normal cells by immuno-assay, which comprises using a monoclonal antibody which is specific to a tissue plasminogen activator obtained from a tissue cultured liquor of human normal tissue derived cells, having the properties of claim 1, and which antibody has the following properties:

a) Molecular weight: 153,000±5,000
b) IgG subclass: IgG1
c) Isoelectric point: 6.6 to 6.9
d) Site of binding to antigen: a fibrin binding site
e) N-terminal amino acid sequence at L Chain and H Chain:

$$\overset{1}{\phantom{}} \qquad\qquad\qquad \overset{10}{\phantom{}}$$

L Chain: Asp-Ile-Lys-Met-Thr-Gln-Ser-Pro-Ser-Ser-

$$\overset{20}{\phantom{}}$$

Met-Tyr-Ala-Ser-Leu-Gly-Glu-Arg-Val-Thr-

$$\overset{30}{\phantom{}}$$

Ile-Thr-Cys-Lys-Ala-Ser-Gln-Asp-Ile-Asn-

$$\overset{40}{\phantom{}} \quad \overset{42}{\phantom{}}$$

Ser-His-Leu-X-Trp-Phe-Gln-Gln-Lys-Pro-Gly-Lys

$$\overset{1}{\phantom{}}$$

H Chain: (Pca)-Ile-Gln-Leu-Val-Gln-Ser-Gly-Pro-

$$\overset{10}{\phantom{}}$$

Glu-Leu-Lys-Lys-Pro-Gly-Glu-Thr-Val-Lys-

$$\overset{20}{\phantom{}}$$

Ile-Ser-Cys-Lys-Ala-Ser-Gly-Tyr-Thr-Phe-

$$\overset{30}{\phantom{}} \qquad \overset{34}{\phantom{}}$$

Thr-Asn-Tyr-Gly-Met

## EP 0 190 711 B1

wherein groups in the parentheses are assumed residual groups; X represents an unidentified residual group; and Pca represents a pyrrolidone carboxylic acid.

**Patentansprüche**

1. Monoklonaler Antikörper, der gegen einen Gewebe-Plasminogen-Aktivator spezifisch ist, der aus einer Gewebekulturflüssigkeit von normalen Human-Gewebe entstammenden Zellen erhalten wurde und die folgenden Eigenschaften besitzt:
   - a) Molekulargewicht: 63 000±10 000
   - b) Isoelektrischer Punkt: 7,0 bis 8,5
   - c) Affinität zu Fibrin: vorhanden
   - d) Affinität zu Concanavalin A: vorhanden
   - e) Optimaler pH: 7 bis 9,5
   - f) Reaktionsfähigkeit gegenüber einem anti-Urokinasespezifischen Antikörper: keine;
   wobei der Antikörper die folgenden Eigenschaften besitzt:
   - a) Molekulargewicht: 150 000±5 000
   - b) IgG-Unterklasse: IgG1
   - c) Isoelektrischer Punkt: 6,7 bis 7,0
   - d) Bindungszentrum des Antigens: Determinante verschieden von einem Fibrin-Bindungszentrum und einem fibrinolytische Aktivität manifestierenden Zentrum
   - e) N-terminale Aminosäure-Sequenzen an der L-Kette und H-Kette:

$$
\begin{array}{ll}
 & \qquad\qquad 1 \qquad\qquad\qquad\qquad\qquad 10 \\
\text{L-Kette:} & \text{Asp-Phe-Val-Met-Thr-Gln-Ser-Pro-Ala-Thr-} \\
\end{array}
$$

$$
\qquad\qquad\qquad\qquad\qquad\qquad\qquad 20 \\
\text{Leu-Ser-Val-Thr-Pro-Gly-Asp-Arg-Val-Ser-}
$$

$$
\qquad\qquad\qquad\qquad\qquad\qquad\qquad 30 \\
\text{Leu-Ser-Cys-Arg-Ala-Ser-Gln-X-Ile-Ser-Asp-}
$$

$$
\qquad\qquad\qquad\qquad\qquad\qquad\qquad 40 \\
\text{Tyr-Leu-His-Trp-Tyr-Gln-Gln-Lys-(Gly)}
$$

$$
\begin{array}{ll}
 & \qquad\qquad 1 \qquad\qquad\qquad\qquad\qquad 10 \\
\text{H-Kette:} & \text{Asp-Val-Gln-Leu-Gln-Gln-Ser-Gly-Pro-Asp-} \\
\end{array}
$$

$$
\qquad\qquad\qquad\qquad\qquad\qquad\qquad 20 \\
\text{Leu-Val-Lys-Pro-Ser-Gln-Ser-Leu-Ser-Leu-}
$$

$$
\qquad\qquad\qquad\qquad\qquad\qquad\qquad 30 \\
\text{Thr-Cys-Thr-Val-Thr-Gly-Tyr-(Ser)-Ile-Thr-X-Gly-Tyr}
$$

worin Gruppen in Klammern als restliche Gruppen angenommen werden und X eine nicht identifizierte restliche Gruppe bezeichnet.

2. Monoklonaler Antikörper nach Anspruch 1, der durch Kultivieren einer Hybridzelle aus Myelom-Zellen von Mäusen und Splenocyten von Mäusen erzeugt wird, die mit Gewebe-Plasminogen-Aktivator immunisiert wurden, der aus einer Gewebekulturflüssigkeit von normalem Human-Gewebe entstammenden Zellen erhalten wurde und die Eigenschaften nach Anspruch 1 besitzt.

3. Monoklonaler Antikörper nach Anspruch 2, worin die Hybridzelle Hybridoma X-21 mit der Bezeichnung IFO 50086 ist.

4. Verfahren zur Reinigung eines aus normalen Human-Zellen stammenden Gewebe-Plasminogen-Aktivators durch Immunoaffinitätschromatographie, umfassend die Verwendung eines monoklonalen Antikörpers, der gegen einen Gewebe-Plasminogen-Aktivator spezifisch ist, der aus einer Gewebekulturflüssigkeit von normalem Human-Gewebe entstammenden Zellen erhalten wurde und die Eigenschaften nach Anspruch 1 besitzt, wobei der Antikörper die folgenden Eigenschaften besitzt:
   - a) Molekulargewicht: 150 000±5 000
   - b) IgG-Unterklasse: IgG1
   - c) Isoelektrischer Punkt: 6,7 bis 7,0
   - d) Bindungszentrum des Antigens: Determinante verschieden von einem Fibrin-Bindungszentrum und einem fibrinolytische Aktivität manifestierenden Zentrum
   - e) N-terminale Aminosäure-Sequenzen an der L-Kette und H-Kette:

```
              1                          10
L-Kette:  Asp-Phe-Val-Met-Thr-Gln-Ser-Pro-Ala-Thr-

                                         20
          Leu-Ser-Val-Thr-Pro-Gly-Asp-Arg-Val-Ser-

                                         30
          Leu-Ser-Cys-Arg-Ala-Ser-Gln-X-Ile-Ser-Asp-

                                         40
          Tyr-Leu-His-Trp-Tyr-Gln-Gln-Lys-(Gly)


              1                          10
H-Kette:  Asp-Val-Gln-Leu-Gln-Gln-Ser-Gly-Pro-Asp-

                                         20
          Leu-Val-Lys-Pro-Ser-Gln-Ser-Leu-Ser-Leu-

                                         30
          Thr-Cys-Thr-Val-Thr-Gly-Tyr-(Ser)-Ile-Thr-X-Gly-Tyr
```

worin Gruppen in Klammern als restliche Gruppen angenommen werden und X eine nicht identifizierte restliche Gruppe bezeichnet.

5. Verfahren zum Nachweis eines aus normalen Human-Zellen stammenden Gewebe-Plasminogen-Aktivators durch Immunoassay, umfassend die Verwendung eines monoklonalen Antikörpers, der gegen einen Gewebe-Plasminogen-Aktivator spezifisch ist, der aus einer Gewebekulturflüssigkeit von normalem Human-Gewebe entstammenden Zellen erhalten wurde und die Eigenschaften nach Anspruch 1 besitzt, wobei der Antikörper die folgenden Eigenschaften besitzt:

a) Molekulargewicht: 150 000±5 000
b) IgG-Unterklasse: IgG1
c) Isoelektrischer Punkt: 6,7 bis 7,0
d) Bindungszentrum des Antigens: Determinante verschieden von einem Fibrin-Bindungszentrum und einem fibrinolytische Aktivität manifestierenden Zentrum
e) N-terminale Aminosäure-Sequenzen an der L-Kette und H-Kette:

```
              1                          10
L-Kette:  Asp-Phe-Val-Met-Thr-Gln-Ser-Pro-Ala-Thr-

                                         20
          Leu-Ser-Val-Thr-Pro-Gly-Asp-Arg-Val-Ser-

                                         30
          Leu-Ser-Cys-Arg-Ala-Ser-Gln-X-Ile-Ser-Asp-

                                         40
          Tyr-Leu-His-Trp-Tyr-Gln-Gln-Lys-(Gly)


              1                          10
H-Kette:  Asp-Val-Gln-Leu-Gln-Gln-Ser-Gly-Pro-Asp-

                                         20
          Leu-Val-Lys-Pro-Ser-Gln-Ser-Leu-Ser-Leu-

                                         30
          Thr-Cys-Thr-Val-Thr-Gly-Tyr-(Ser)-Ile-Thr-X-Gly-Tyr
```

worin Gruppen in Klammern als restliche Gruppen angenommen werden und X eine nicht identifizierte restliche Gruppe bezeichnet.

6. Monoklonaler Antikörper, der gegen einen Gewebe-Plasminogen-Aktivator spezifisch ist, der aus einer Gewebekulturflüssigkeit von normalen Human-Gewebe entstammenden Zellen erhalten wurde und die Eigenschaften nach Anspruch 1 besitzt, wobei der Antikörper die folgenden Eigenschaften besitzt:

a) Molekulargewicht: 153 000±5 000
b) IgG-Unterklasse: IgG1

c) Isoelektrischer Punkt: 6,6 bis 6,9
d) Bindungszentrum des Antigens: Ein Fibrin-Bindungszentrum
e) N-terminale Aminosäure-Sequenzen an der L-Kette und H-Kette:

```
                    1                            10
L-Kette:  Asp-Ile-Lys-Met-Thr-Gln-Ser-Pro-Ser-Ser-

                                             20
          Met-Tyr-Ala-Ser-Leu-Gly-Glu-Arg-Val-Thr-

                                             30
          Ile-Thr-Cys-Lys-Ala-Ser-Gln-Asp-Ile-Asn-

                                         40        42
          Ser-His-Leu-X-Trp-Phe-Gln-Gln-Lys-Pro-Gly-Lys


                    1
H-Kette:  (Pca)-Ile-Gln-Leu-Val-Gln-Ser-Gly-Pro-

                   10
          Glu-Leu-Lys-Lys-Pro-Gly-Glu-Thr-Val-Lys-

                   20
          Ile-Ser-Cys-Lys-Ala-Ser-Gly-Tyr-Thr-Phe-

                   30              34
          Thr-Asn-Tyr-Gly-Met
```

worin Gruppen in Klammern als restliche Gruppen angenommen werden, X eine nicht identifizierte restliche Gruppe bezeichnet und Pca einen Pyrrolidincarbonsäure-Rest bezeichnet.

7. Monoklonaler Antikörper nach Anspruch 6, der durch Kultivieren einer Hybridzelle aus Myelom-Zellen von Mäusen und Splenocyten von Mäusen erzeugt wird, die mit Gewebe-Plasminogen-Aktivator immunisiert wurden, der aus einer Gewebekulturflüssigkeit von normalem Human-Gewebe entstammenden Zellen erhalten wurde und die Eigenschaften nach Anspruch 1 besitzt.

8. Monoklonaler Antikörper nach Anspruch 7, worin die Hybridzelle Hybridoma X-23 mit der Bezeichnung IFO 50887 ist.

9. Verfahren zur Reinigung eines aus normalen Human-Zellen stammenden Gewebe-Plasminogen-Aktivators durch Immunoaffinitätschromatographie, umfassend die Verwendung eines monoklonalen Antikörpers, der gegen einen Gewebe-Plasminogen-Aktivator spezifisch ist, der aus einer Gewebekulturflüssigkeit von normalem Human-Gewebe entstammenden Zellen erhalten wurde und die Eigenschaften nach Anspruch 1 besitzt, wobei der Antikörper die folgenden Eigenschaften besitzt:
a) Molekulargewicht: 153 000±5 000
b) IgG-Unterklasse: IgG1
c) Isoelektrischer Punkt: 6,6 bis 6,9
d) Bindungszentrum des Antigens: Ein Fibrin-Bindungszentrum
e) N-terminale Aminosäure-Sequenzen an der L-Kette und H-Kette:

```
                    1                            10
L-Kette:  Asp-Ile-Lys-Met-Thr-Gln-Ser-Pro-Ser-Ser-

                                             20
          Met-Tyr-Ala-Ser-Leu-Gly-Glu-Arg-Val-Thr-

                                             30
          Ile-Thr-Cys-Lys-Ala-Ser-Gln-Asp-Ile-Asn-

                                         40        42
          Ser-His-Leu-X-Trp-Phe-Gln-Gln-Lys-Pro-Gly-Lys
```

# EP 0 190 711 B1

H-Kette: (Pca)-Ile-Gln-Leu-Val-Gln-Ser-Gly-Pro-

10
Glu-Leu-Lys-Lys-Pro-Gly-Glu-Thr-Val-Lys-

20
Ile-Ser-Cys-Lys-Ala-Ser-Gly-Tyr-Thr-Phe-

30    34
Thr-Asn-Tyr-Gly-Met

worin Gruppen in Klammern als restliche Gruppen angenommen werden, X eine nicht identifizierte restliche Gruppe bezeichnet und Pca einen Pyrrolidincarbonsäure-Rest bezeichnet.

10. Verfahren zum Nachweis eines aus normalen Human-Zellen stammenden Gewebe-Plasminogen-Aktivators durch Immunoassay, umfassend die Verwendung eines monoklonalen Antikörpers, der gegen einen Gewebe-Plasminogen-Aktivator spezifisch ist, der aus einer Gewebekulturflüssigkeit von normalen Human-Gewebe entstammenden Zellen erhalten wurde und die Eigenschaften nach Anspruch 1 besitzt, wobei der Antikörper die folgenden Eigenschaften besitzt:

a) Molekulargewicht: 153 000±5 000
b) IgG-Unterklasse: IgG1
c) Isoelektrischer Punkt: 6,6 bis 6,9
d) Bindungszentrum des Antigens: Ein Fibrin-Bindungszentrum
e) N-terminale Aminosäure-Sequenzen an der L-Kette und H-Kette:

1                        10
L-Kette:  Asp-Ile-Lys-Met-Thr-Gln-Ser-Pro-Ser-Ser-

20
Met-Tyr-Ala-Ser-Leu-Gly-Glu-Arg-Val-Thr-

30
Ile-Thr-Cys-Lys-Ala-Ser-Gln-Asp-Ile-Asn-

40    42
Ser-His-Leu-X-Trp-Phe-Gln-Gln-Lys-Pro-Gly-Lys

1
H-Kette:  (Pca)-Ile-Gln-Leu-Val-Gln-Ser-Gly-Pro-

10
Glu-Leu-Lys-Lys-Pro-Gly-Glu-Thr-Val-Lys-

20
Ile-Ser-Cys-Lys-Ala-Ser-Gly-Tyr-Thr-Phe-

30    34
Thr-Asn-Tyr-Gly-Met

worin Gruppen in Klammern als restliche Gruppen angenommen werden, X eine nicht identifizierte restliche Gruppe bezeichnet und Pca einen Pyrrolidincarbonsäure-Rest bezeichnet.

**Revendications**

1. Anticorps monoclonal spécifique d'un activateur de plasminogène tissulaire obtenu à partir d'un liquide de culture tissulaire de cellules dérivées d'un tissu humain normal, ayant les propriétés suivantes:
a) poids moléculaire: 63 000±10 000
b) point isoélectrique: 7,0 à 8,5
c) affinité pour la fibrine: présente
d) affinité pour la concanavaline A: présente
e) pH optimal: 7 à 9,5
f) réactivité avec un anticorps spécifique anti-urokinase: néant lequel anticorps a les propriétés suivantes:
a) poids moléculaire: 150 000±5 000

b) sous-classe d'IgG: IgG1

c) point isoélectrique: 6,7 à 7,0

d) site de fixation à un antigène: déterminant autre qu'un site de liaison de la fibrine et un site manifestant une activité fibrinolytique

e) séquence N-terminale des amino-acides de la chaîne L et de la chaîne H:

```
                1                                    10
     chaîne L:  Asp-Phe-Val-Met-Thr-Gln-Ser-Pro-Ala-Thr-


                                                     20
                Leu-Ser-Val-Thr-Pro-Gly-Asp-Arg-Val-Ser-


                                                     30
                Leu-Ser-Cys-Arg-Ala-Ser-Gln-X-Ile-Ser-Asp-


                                                     40
                Tyr-Leu-His-Trp-Tyr-Gln-Gln-Lys-(Gly)



                1                                    10
     chaîne H:  Asp-Val-Gln-Leu-Gln-Gln-Ser-Gly-Pro-Asp-


                                                     20
                Leu-Val-Lys-Pro-Ser-Gln-Ser-Leu-Ser-Leu-


                                                     30
                Thr-Cys-Thr-Val-Thr-Gly-Tyr-(Ser)-Ile-Thr-X-Gly-Tyr
```

où les groupes entre parenthèses sont des groupes résiduels supposés; et X représente un groupe résiduel non identifié.

2. Anticorps monoclonal selon la revendication 1 qui est produit par culture d'une cellule hybride de cellules de myélome de souris et de splénocytes de souris immunisées avec un activateur de plasminogène tissulaire obtenu à partir d'un liquide de culture tissulaire de cellules dérivées d'un tissu humain normal, ayant les propriétés indiquées dans la revendication 1.

3. Anticorps monoclonal selon la revendication 2 dans lequel ladite cellule hybride est l'hybridome X-21 IFO 50086.

4. Procédé pour purifier un activateur de plasminogène tissulaire dérivé de cellules humaines normales par chromatographie d'immuno-affinité, qui comprend l'utilisation d'un anticorps monoclonal qui est spécifique d'un activateur de plasminogène tissulaire obtenu à partir d'un liquide de culture tissulaire de cellules dérivées d'un tissu humain normal, ayant les propriétés indiquées dans la revendication 1, lequel anticorps a les propriétés suivantes:

a) poids moléculaire: 150 000±5 000

b) sous-classe d'IgG: IgG1

c) point isoélectrique: 6,7 à 7,0

d) site de fixation à un antigène: déterminant autre qu'un site de liaison de la fibrine et un site manifestant une activité fibrinolytique

e) séquence N-terminale des amino-acides de la chaîne L et de la chaîne H:

```
                1                                    10
     chaîne L:  Asp-Phe-Val-Met-Thr-Gln-Ser-Pro-Ala-Thr-


                                                     20
                Leu-Ser-Val-Thr-Pro-Gly-Asp-Arg-Val-Ser-


                                                     30
                Leu-Ser-Cys-Arg-Ala-Ser-Gln-X-Ile-Ser-Asp-


                                                     40
                Tyr-Leu-His-Trp-Tyr-Gln-Gln-Lys-(Gly)
```

**EP 0 190 711 B1**

```
              1                              10
chaîne  H:  Asp-Val-Gln-Leu-Gln-Gln-Ser-Gly-Pro-Asp-

                                              20
            Leu-Val-Lys-Pro-Ser-Gln-Ser-Leu-Ser-Leu-

                                              30
            Thr-Cys-Thr-Val-Thr-Gly-Tyr-(Ser)-Ile-Thr-X-Gly-Tyr
```

où les groupes entre parenthèses sont des groupes résiduels supposés; et X représente un groupe résiduel non identifié.

5. Procédé pour la détection par immunodétermination d'un activateur de plasminogène tissulaire dérivé de cellules humaines normales, qui comprend l'utilisation d'un anticorps monoclonal qui est spécifique d'un activateur de plasminogène tissulaire obtenu à partir d'un liquide de culture tissulaire de cellules dérivées d'un tissu humain normal, ayant les propriétés indiquées dans la revendication 1, lequel anticorps a les propriétés suivantes:

a) poids moléculaire: 150 000±5 000
b) sous-classe d'IgG: IgG1
c) point isoélectrique: 6,7 à 7,0
d) site de fixation à un antigène: déterminant autre qu'un site de liaison de la fibrine et un site manifestant une activité fibrinolytique
e) séquence N-terminale des amino-acides de la chaîne L et de la chaîne H:

```
              1                              10
chaîne  L:  Asp-Phe-Val-Met-Thr-Gln-Ser-Pro-Ala-Thr-

                                              20
            Leu-Ser-Val-Thr-Pro-Gly-Asp-Arg-Val-Ser-

                                              30
            Leu-Ser-Cys-Arg-Ala-Ser-Gln-X-Ile-Ser-Asp-

                                              40
            Tyr-Leu-His-Trp-Tyr-Gln-Gln-Lys-(Gly)


              1                              10
chaîne  H:  Asp-Val-Gln-Leu-Gln-Gln-Ser-Gly-Pro-Asp-

                                              20
            Leu-Val-Lys-Pro-Ser-Gln-Ser-Leu-Ser-Leu-

                                              30
            Thr-Cys-Thr-Val-Thr-Gly-Tyr-(Ser)-Ile-Thr-X-Gly-Tyr
```

où les groupes entre parenthèses sont des groupes résiduels supposés; et X représente un groupe résiduel non identifié.

6. Anticorps monoclonal qui est spécifique d'un activateur de plasminogène tissulaire obtenu à partir d'un liquide de culture tissulaire de cellules dérivées d'un tissu humain normal ayant les propriétés indiquées dans la revendication 1, lequel anticorps a les propriétés suivantes:

a) poids moléculaire: 153 000±5 000
b) sous-classe d'IgG: IgG1
c) point isoélectrique: 6,6 à 6,9
d) site de fixation à un antigène: un site de fixation de la fibrine
e) séquence N-terminale des amino-acides de la chaîne L et de la chaîne H:

```
              1                              10
chaîne  L:  Asp-Ile-Lys-Met-Thr-Gln-Ser-Pro-Ser-Ser-

                                              20
            Met-Tyr-Ala-Ser-Leu-Gly-Glu-Arg-Val-Thr-

                                              30
            Ile-Thr-Cys-Lys-Ala-Ser-Gln-Asp-Ile-Asn-

                                              40        42
            Ser-His-Leu-X-Trp-Phe-Gln-Gln-Lys-Pro-Gly-Lys
```

chaîne H: (Pca)-Ile-Gln-Leu-Val-Gln-Ser-Gly-Pro-

Glu-Leu-Lys-Lys-Pro-Gly-Glu-Thr-Val-Lys-

Ile-Ser-Cys-Lys-Ala-Ser-Gly-Tyr-Thr-Phe-

Thr-Asn-Tyr-Gly-Met

où les groupes entre parenthèses sont des groupes résiduels supposés; X représente un groupe résiduel non identifié; et Pca représente un acide pyrrolidonecarboxylique.

7. Anticorps monoclonal selon la revendication 6 qui est produit par culture d'une cellule hybride de cellules de myélome de souris et de splénocytes de souris immunisées avec un activateur de plasminogène tissulaire obtenu à partir d'un liquide de culture tissulaire de cellules dérivées d'un tissu humain normal, ayant les propriétés indiquées dans la revendication 1.

8. Anticorps monoclonal selon la revendication 7 où ladite cellule hybride est l'hybridome X-23 IFO 50087.

9. Procédé pour purifier par chromatographie d'immuno-affinité un activateur de plasminogène tissulaire dérivé de cellules humaines normales, qui comprend l'utilisation d'un anticorps monoclonal qui est spécifique d'un activateur de plasminogène tissulaire obtenu à partir d'un liquide de culture tissulaire de cellules dérivées d'un tissu humain normal, ayant les propriétés indiquées dans la revendication 1, lequel anticorps à les propriétés suivantes:

    a) poids moléculaire: 153 000±5 000
    b) sous-classe d'IgG: IgG1
    c) point isoélectrique: 6,6 à 6,9
    d) site de fixation à un antigène: un site de fixation de la fibrine
    e) séquence N-terminale des amino-acides de la chaîne L et de la chaîne H:

chaîne L: Asp-Ile-Lys-Met-Thr-Gln-Ser-Pro-Ser-Ser-

Met-Tyr-Ala-Ser-Leu-Gly-Glu-Arg-Val-Thr-

Ile-Thr-Cys-Lys-Ala-Ser-Gln-Asp-Ile-Asn-

Ser-His-Leu-X-Trp-Phe-Gln-Gln-Lys-Pro-Gly-Lys

chaîne H: (Pca)-Ile-Gln-Leu-Val-Gln-Ser-Gly-Pro-

Glu-Leu-Lys-Lys-Pro-Gly-Glu-Thr-Val-Lys-

Ile-Ser-Cys-Lys-Ala-Ser-Gly-Tyr-Thr-Phe-

Thr-Asn-Tyr-Gly-Met

où les groupes entre parenthèses sont des groupes résiduels supposés; X représente un groupe résiduel non identifié; et Pca représente un acide pyrrolidonecarboxylique.

10. Procédé pour la détection par immunodétermination d'un activateur de plasminogène tissulaire dérivé de cellules humaines normales, qui comprend l'utilisation d'un anticorps monoclonal qui est spécifique d'un activateur de plasminogène tissulaire obtenu à partir d'un liquide de culture tissulaire de cellules dérivées d'un tissu humain normal, ayant les propriétés indiquées dans la revendication 1, lequel anticorps a les propriétés suivantes:

    a) poids moléculaire: 153 000±5 000
    b) sous-classe d'IgG: IgG1
    c) point isoélectrique: 6,6 à 6,9

d) site de fixation à un antigène: un site de fixation de la fibrine

e) séquence N-terminale des amino-acides de la chaîne L et de la chaîne H:

<div align="center">

1         10

chaîne L: Asp-Ile-Lys-Met-Thr-Gln-Ser-Pro-Ser-Ser-

20

Met-Tyr-Ala-Ser-Leu-Gly-Glu-Arg-Val-Thr-

30

Ile-Thr-Cys-Lys-Ala-Ser-Gln-Asp-Ile-Asn-

40  42

Ser-His-Leu-X-Trp-Phe-Gln-Gln-Lys-Pro-Gly-Lys

1

chaîne H: (Pca)-Ile-Gln-Leu-Val-Gln-Ser-Gly-Pro-

10

Glu-Leu-Lys-Lys-Pro-Gly-Glu-Thr-Val-Lys-

20

Ile-Ser-Cys-Lys-Ala-Ser-Gly-Tyr-Thr-Phe-

30    34

Thr-Asn-Tyr-Gly-Met

</div>

où les groupes entre parenthèses sont des groupes résiduels supposés; X représente un groupe résiduel non identifié; et Pca représente un acide pyrrolidonecarboxylique.

## FIG.1a

EP 0 190 711 B1

FIG.1b

FIG.2a

FIG.2b

FIG.3

FIG.4